# EUROPÄISCHE PATENTSCHRIFT

(11) **EP 2 630 990 B1**
(45) Veröffentlichungstag und Bekanntmachung des Hinweises auf die Patenterteilung: **11.04.2018**
(21) Anmeldenummer: 12198440.5
(22) Anmeldetag: 20.12.2012
(51) Int. Cl.: A61Q 17/04, A61K 8/37, A61K 8/35, A61K 8/06, A61K 8/44

(54) **Leichte, wasserfeste kosmetische Zubereitung**
Lightweight, waterproof cosmetic preparation
Préparation cosmétique légère et résistante à l'eau

(30) Priorität: 04.01.2012 DE 102012200074
(43) Veröffentlichungstag der Anmeldung: 28.08.2013
(73) Patentinhaber: Beiersdorf AG, 20253 Hamburg (DE)
(72) Erfinder: Zanforlin Trede, Lucia, 22529 Hamburg (DE); Thilker, Janina, 22303 Hamburg (DE); Skubsch, Kerstin, 25497 Prisdorf (DE); Möllgaard, Svenja Lena, 22337 Hamburg (DE); Nielsen, Jens, 22529 Hamburg (DE); Pasternak, Anja, 22085 Hamburg (DE)

(56) Entgegenhaltungen:
- EP-A1- 1 535 938
- WO-A1-2009/018975
- WO-A1-2009/138485
- FR-A1- 2 882 651

## Beschreibung

Die vorliegende Erfindung betrifft eine kosmetische Zubereitung enthaltend 4-(tert.-Butyl)-4'-methoxydibenzoylmethan, Ethylhexylsalicylat, Homomenthylsalicylat (INCI: Homosalate) und einen hydrophilen Emulgator mit einem HLB-Wert von größer oder gleich 15.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Aufgrund ihrer gegenüber W/O-Emulsionen angenehmeren Sensorik und der Tatsache, dass sich mit ihnen generell höhere Lichtschutzfaktoren erzielen lassen, werden als Grundlage für Sonnenschutzmittel und Tagespflegeprodukte bevorzugt ÖI-in-Wasser-Emulsionen (O/W-Emulsionen) eingesetzt. O/W-Emulsionen haben jedoch aufgrund der wässrigen äußeren Phase den Nachteil, nicht besonders wasserfest zu sein, wobei die Wasserfestigkeit generell mit zunehmenden HLB-Wert des eingesetzten Emulgators abnimmt. Da aber Sonnenschutzmittel häufig am Strand oder Schwimmbad zum Einsatz kommen, also bei Aktivitäten, bei denen die Anwender mehr oder weniger intensiv mit Wasser in Kontakt kommen, besteht ein großer Bedarf an "wasserfesten" Sonnenschutzmitteln. Nach herkömmlichen Stand der Technik, wird versucht, die Wasserfestigkeit von Sonnenschutzmitteln, insbesondere auf O/W-Emulsionsbasis, dadurch zu erhöhen, dass man den Zubereitungen sogenannte Filmbildner zusetzt, die bei der Applikation auf der Haut, die UV-Filtersubstanzen fixieren sollen. Nachteilig an diesen Zubereitungen ist jedoch der Umstand, dass diese Filmbildner dazu führen, dass die Zubereitungen ein klebrig-schmieriges Hautgefühl verursachen, also sensorisch unattraktiv sind. Darüber hinaus führen diese Filmbildner dazu, dass die Sandanhaftung (oder Anhaftung anderer "Schmutzpartikel") auf der Haut erhöht wird.

Es war daher die Aufgabe der vorliegenden Erfindung, die Wasserfestigkeit von Sonnenschutzmitteln (und Tagespflegeprodukten) auf O/W-Emulsionsbasis auf der Haut zu verbessern, ohne dass die Zubereitungen ein klebrig-schmieriges Hautgefühl erzeugen und die Neigung zur Anhaftung von Sand oder anderen Partikeln reduziert ist.

Ein weiterer Nachteil des Standes der Technik besteht in dem Umstand, dass sich in O/W Emulsionen, die mit einem Emulgator mit hohem HLB-Wert gebildet werden nur schwer größere Mengen an lipophilen UV-Filtern einarbeiten lassen ohne dass die Stabilität (Langzeitstabilität, Temperaturstabilität, Lagerstabilität) der Emulsion darunter leidet. Insbesondere bei höheren Gehalten an 4-(tert.-Butyl)-4'-methoxydibenzoylmethan, Ethylhexylsalicylat und Homomenthylsalicylat (INCI: Homosalate) sind die Einsatzkonzentrationen bei Emulgatoren mit hohem HLB-Wert begrenzt.

Es war daher die Aufgabe der vorliegenden Erfindung, eine stabile (langzeitstabile, lagerstabile, temperaturstabile) O/W-Emulsion mit einem hohen Gehalt an an 4-(tert.-Butyl)-4'-methoxydibenzoylmethan, Ethylhexylsalicylat und Homomenthylsalicylat (INCI: Homosalate) zu entwickeln.

Überraschend gelöst werden die Aufgaben durch eine kosmetische Öl-in-Wasser Emulsion (O/W Emulsion) enthaltend
a) 4-(tert.-Butyl)-4'-methoxydibenzoylmethan in einer Gesamtkonzentration von 2 bis 5 Gewichts-%, bezogen auf das Gesamtgewicht der Zubereitung,
b) Ethylhexylsalicylat in einer Gesamtkonzentration von 2 bis 5 Gewichts-%, bezogen auf das Gesamtgewicht der Zubereitung,
c) Homomenthylsalicylat (INCI: Homosalate) in einer Gesamtkonzentration von 3 bis 10 Gewichts-%, bezogen auf das Gesamtgewicht der Zubereitung und
d) Natriumstearoylglutamat (INCI Sodium Stearoyl Glutamate).

Die erfindungsgemäßen Zubereitungen weisen ein überraschend leichtes Hautgefühl auf, sind nicht klebrig und erstaunlich wasserfest.

Zwar kennt der Stand der Technik die DE 102007024345, DE 102008028665, DE 102007038413, DE 102007024342 und DE 102007017439, doch konnten diese Schriften nicht den Weg zur vorliegenden Erfindung weisen.

Ferner kennt der Stand der Technik die Dokumente EP 1535938, WO 2009/138485, WO 2009/018975, FR 2882651 und WO 2011/063329, die ebenfalls nicht den Weg zur vorliegenden Erfindung weisen konnten.

Es ist erfindungsgemäß vorteilhaft, wenn die Zubereitung Natriumstearoylglutamat (INCI Sodium Stearoyl Glutamate) in einer Gesamtkonzentration von 0,15 bis 0,75 Gewichts-%, bezogen auf das Gesamtgewicht der Zubereitung, enthält.

Es ist erfindungsgemäß bevorzugt, wenn die Zubereitung Natriumstearoylglutamat (INCI Sodium Stearoyl Glutamate) in einer Gesamtkonzentration von 0,25 bis 0,5 Gewichts-%, bezogen auf das Gesamtgewicht der Zubereitung, enthält.

Es ist besonders bevorzugt im Sinne der vorliegenden Erfindung, wenn die Zubereitung 4-(tert.-Butyl)-4'-methoxydibenzoylmethan in einer Gesamtkonzentration von 3,5 bis 5 Gewichts-%, bezogen auf das Gesamtgewicht der Zubereitung, enthält.

Erfindungsgemäß besonders bevorzugte Ausführungsformen der vorliegenden Erfindung sind dadurch gekennzeichnet, dass die Zubereitung Ethylhexylsalicylat in einer Gesamtkonzentration von 3,5 bis 5 Gewichts-%, bezogen auf das Gesamtgewicht der Zubereitung, enthält.

Es ist erfindungsgemäß besonders bevorzugt, wenn die erfindungsgemäße Zubereitung Homomenthylsalicylat in einer Gesamtkonzentration von 6 bis 10 Gewichts-%, bezogen auf das Gesamtgewicht der Zubereitung, enthält.

Es ist vorteilhaft im Sinne der vorliegenden Erfindung, wenn die Zubereitung Ethanol enthält.

Dabei ist es erfindungsgemäß bevorzugt, wenn die Zubereitung Ethanol in einer Konzentration von 2 bis 10 Gewichts-%, bezogen auf das Gesamtgewicht der Zubereitung, enthält.

Es ist erfindungsgemäß besonders bevorzugt, wenn die Zubereitung Ethanol in einer Konzentration von 4 bis 8 Gewichts-%, bezogen auf das Gesamtgewicht der Zubereitung, enthält.

Erfindungsgemäß vorteilhaft enthält die erfindungsgemäße Zubereitung einen oder mehrere Parfümstoffe.

Erfindungsgemäß vorteilhafte Ausführungsformen der vorliegenden Erfindung sind dadurch gekennzeichnet, dass der oder die erfindungsgemäßen Parfümstoffe gewählt werden aus der Gruppe Linalool, Benzyl Benzoate, Hydroxyisohexyl 3-Cyclohexene Carboxaldehyd, Hexyl Cinnamal, Benzyl Salicylate, Citronellol, Coumarin, Limonen, Butylphenyl Methylpropional, Eugenol, Geraniol, Alpha-Isomethyl lonone.

Dabei ist es erfindungsgemäß bevorzugt, wenn die erfindungsgemäßen Parfümstoffe ausgewählt werden aus der Gruppe der Verbindungen Coumarin, Limonen, Eugenol und Geraniol.

Es ist erfindungsgemäß bevorzugt, wenn die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)ben- zolsulfonsäuresalze; 2-Methyl-5-(2-oxo-3-bornylidenmethyl)sulfonsäuresalze; 2,2'-Methylen- bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat;; Dimethicodiethylbenzalmalonat; 3-(4-(2,2-bis Ethoxycarbonylvinyl)-phenoxy)propenyl)-meth-oxysiloxan / Dimethylsiloxan - Copolymer; ) 2-Ethylhexyl-2-cyano-3,3-diphenylacrylat (Octocrylen); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethyl-hexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

Die erfindungsgemäß vorteilhaften Merocyanine werden dabei gewählt aus der Gruppe der Verbindungen 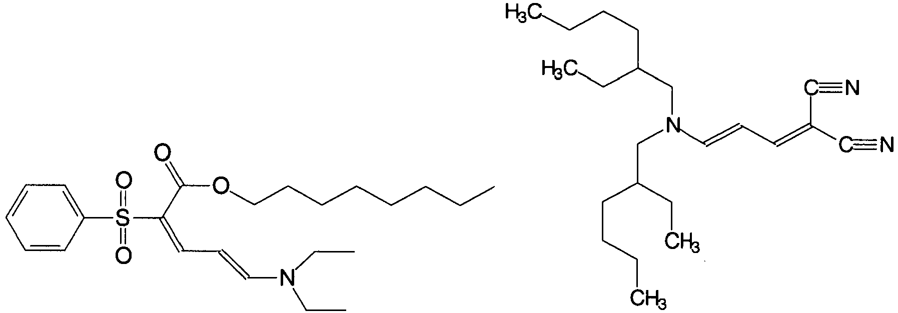 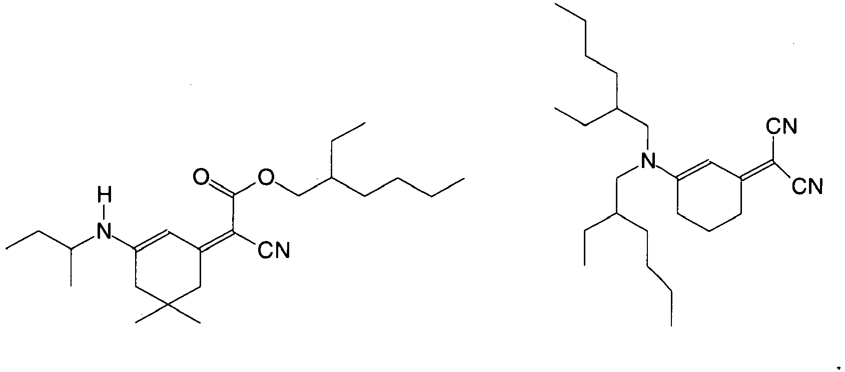 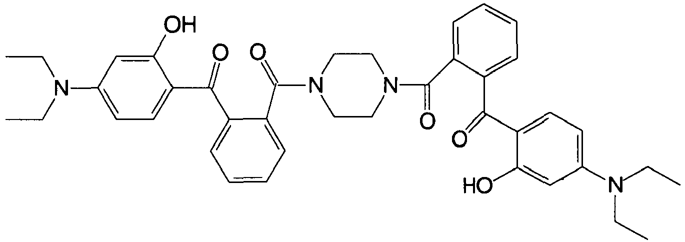 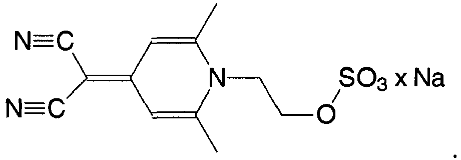 Als erfindungsgemäß vorteilhaftes Piperazinderivat kann dabei die folgende Verbindung eingesetzt werden: 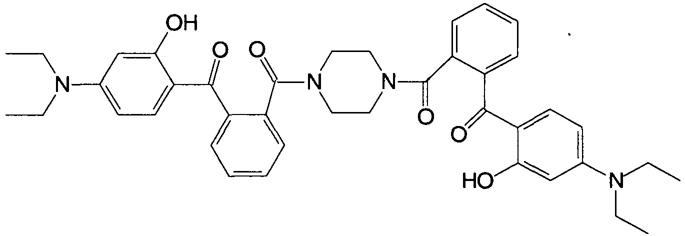 Es ist erfindungsgemäß bevorzugt, wenn die erfindungsgemäße Zubereitung frei ist von 4-Methoxyzimtsäure(2-ethylhexyl)ester (INCI: Ethylhexyl Methoxycinnamate) und p-Methylbenzylidencampher.

Erfindungsgemäß bevorzugte Ausführungsformen der vorliegenden Erfindung sind ferner dadurch gekennzeichnet, dass die Zubereitung einen Lichtschutzfaktor (SPF) von größer oder gleich 30 aufweist.

Erfindungsgemäß vorteilhafte Ausführungsformen der vorliegenden Erfindung sind dadurch gekennzeichnet, dass die erfindungsgemäße Zubereitung Glycerin enthält.

Es ist erfindungsgemäß vorteilhaft, wenn die erfindungsgemäße Zubereitung Feuchthaltemittel wie Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure, Hyaluronsäure, Chitosan und/oder Harnstoff enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum.

Es ist erfindungsgemäß bevorzugt, wenn die Zubereitung Xanthangummi und/oder Tapiokastärke enthält.

Es ist erfindungsgemäß bevorzugt, wenn die Zubereitung ein oder mehrere Diole gewählt aus der Gruppe der Verbindungen 1,2-Pentandiol, 1,2-Hexandiol, 1,2-Octandiol, 1,2-Decandiol, 2-Methylpropan-1,3-diol enthält.
Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.
Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).
Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.
Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.
Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.
Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, **C13-16 Isoparaffin** und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Erfindungsgemäß besonders bevorzugte Ausführungsformen der vorliegenden Erfindung sind dadurch gekennzeichnet, dass die Zubereitung eine oder mehrere lipophile Bestandteile gewählt aus der Gruppe der Verbindungen Myristylmyristat, Octyldodecanol, C₁₂-C₁₅-Alkylbenzoat, Butylenglycoldicaprylat/Dicaprat, Ceatylalkohol enthält.

Es ist erfindungsgemäß bevorzugt, wenn die erfindungsgemäße Zubereitung Tocopherylacetat und/oder Tocopherol enthält.
Erfindungsgemäß bevorzugte Ausführungsformen der vorliegenden Erfindung sind dadurch gekennzeichnet, dass die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, ß-Alanin und/oder Licochalcon A, enthält.

Die erfindungsgemäßen Emulsionen können erfindungsgemäß vorteilhaft Methylparaben, Ethylparaben, 2-Methylisothiazol-3(2*H*)-on und/oder Phenoxyethanol enthalten.

Eine alternative Ausführungsform der vorliegenden Erfindung ist jedoch auch dadurch gekennzeichnet, dass die Emusion frei ist von Parabenen.

Es ist erfindungsgemäß vorteilhaft, wenn die erfindungsgemäße Emulsion frei ist von 3-lodo-2-propynylbutylcarbamat.

Vorteilhaft im Sinne der vorliegenden Erfindung sind Zubereitungen zur Pflege der Haut. Sie können dem kosmetischen Lichtschutz, der Hautpflege (beispielsweise zur Prophylaxe und Behandlung der Hautalterung) und dekorativen Kosmetik dienen.

Die Zubereitungen können sprühbar oder auf einem Träger (z.B. Tuch) dargereicht werden.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

### Vergleichsversuch

Der erfinderische Effekt konnte mit dem folgenden Vergleichsversuch belegt werden:

Die beiden Formulierungen wurden nach der Wasserfestigkeit und Sensorik untersucht.

**Rezepturvergleich**

| | **Produkt 1** | **Produkt 2** |
|---|---|---|
| **INCl-Name(n)** | **m [%]** | **m [%]** |
| **Butyl Methoxydibenzoylmethane** | **4.5000** | **4.5000** |
| **Ethylhexyl Salicylate** | **4.5000** | **4.5000** |
| **Homosalate** | **9.0000** | 9.0000 |
| Weitere UV-Filter | 11.0000 | 11.0000 |
| Komplexbildner | 0.2000 | 0.2000 |
| Antioxidant | 0.0600 | 0.0600 |
| Moisturizer | 0.9000 | 0.9000 |
| Füllstoff | 1.0000 | 1.0000 |
| Parfum | 0.4000 | 0.4000 |
| Alcohol Denat. | 8.0000 | 8.0000 |
| Konservierungsmittel | 0.9000 | 0.9000 |
| Verdicker | 0.4000 | 0.4000 |
| Strukturgeber | 1.0000 | 1.0000 |
| Co-Emulqator | 1.0000 | 1.0000 |
| **Sodium Stearoyl Glutamate** | **0.3500** | - |
| **Potassium Cetyl Phosphate** | - | **0.3500** |
| Aqua | Ad 100,0 | Ad 100,0 |

### a) Untersuchung der Wasserfestigkeit

### Durchführung

Die Methode zur Bestimmung der Wasserfestigkeit von Sonnenschutzmitteln ist in den im Dezember 2005 von COLIPA veröffentlichten Leitlinien "Guidelines for Evaluating Sun Product Water Resistance" beschrieben.
Der an einem Probanden vor oder nach dem Eintauchen in Wasser bestimmte Lichtschutzfaktor (LSF) eines Sonnenschutzmittels ist definiert als das Verhältnis der minimalen Erythemdosis (MED) auf der geschützten Haut zur MED auf der ungeschützten Haut des gleichen Probanden. LSF und MED werden nach der "Internationalen Methode zur Bestimmung des Lichtschutzfaktors (LSF)" bestimmt. Alle Bestimmungen des LSF vor und nach dem Eintauchen in Wasser müssen im gleichen Labor mit den gleichen Probanden in der gleichen Testsequenz durchgeführt werden.

### Ergebnisse:

Die Untersuchung nach der Wasserfestigkeit wurde bei einem unabhängigen Institut durchgeführt.

| | **Mean Value** | |
|---|---|---|
| | **LSF** | **Water Resistence [%]** |
| **Produkt 1** | **38** | **71%** |
| **Produkt 2** | **41** | **55%** |

### b) Untersuchung der Sensorik

### Durchführung

Die Studie wurde mit 50 qualifizierten weiblichen und männlichen Testteilnehmern im Alter zwischen 18 und 60 Jahren, die Urlaub machen und ihre Zeit hauptsächlich am Strand verbringen, durchgeführt. Sie erhielten ein Produktmuster pro Code. Die Produkte wurden je vier Tage lang und in dieser Zeit mindestens einmal täglich verwendet. Die Verwendung der Produkte erfolgte sequentiell und in rotierter Reihenfolge, um Positionseffekte zu kontrollieren. Am Ende jeder Testperiode war ein produktspezifischer Fragebogen auszufüllen. Nach Abschluss der Testphase waren zusätzlich einige allgemeine Fragen zum Test zu beantworten. Alle Daten wurden in ein Excel-File übertragen. Die Analyse signifikanter Unterschiede erfolgte mittels Friedmann-Analyse sowie paarweisen post-hoc Wilcoxon-Tests für gepaarte Stichproben.

### Ergebnisse:

Das Produkt 1 kann sich hinsichtlich folgender Statements signifikant von Produkt 2 absetzen:
- Das Produkt fühlt sich leicht an
- Die Haut ist nicht klebrig
- Das Produkt gibt ein leichtes Hautgefühl

### Beispiele

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

| | **1** | **2** | **3** | **4** | **5** | **6** | **7** | **8** |
|---|---|---|---|---|---|---|---|---|
| Acrylat/C10-30 Alkyl Acrylat Crosspolymer | | 0,2 | | 0,2 | | | 0,1 | 0,05 |
| Carbomer | | | | 0,2 | | | | |
| Xanthan Gummi | 0,2 | | 0,3 | | 0,4 | 0,3 | | 0,4 |
| Tapioca Stärke | 1 | | | | | | | |
| Distarch Phosphate | | | 1 | | | | | |
| Carrageenan | | 0,2 | | 0,3 | | | 0,2 | |
| VP/Hexadecene Copolymer | 1,5 | 1 | | 0,5 | 0,5 | 0,5 | 1 | 0,75 |
| Acrylates/C12-22 Alkylmethacrylate Copolymer | | | 1,5 | | | | | 1 |
| Natrium Stearoylglutamat | 0,15 | 0,2 | 0,3 | 0,3,5 | 0,4 | 0,5 | 0,35 | 0,55 |
| Glyceryl Stearate | | 0,5 | 1 | | 0,5 | | | |
| Myristyl Myristat | 1 | 1 | | | | 0,5 | | 0,5 |
| Butylenglycol Dicaprylat/Dicaprat | 1,5 | | 3 | | | | 3 | |
| C12-15 Alkyl Benzoat | | | | 2 | 3 | 2 | | |
| Caprylic/Capric Triglycerid | | | | 2 | | | | |
| Cetearylalkohol | | | | 1 | | 1,5 | | 0,5 |
| Cetyl Alkohol | 2 | | | | | | 2 | 1 |
| Dicaprylylcarbonat | | 1 | | | 0,5 | | | |
| Dimethicon | | | | | | 1 | 1 | |
| Octyldodekanol | 1 | 2 | 3 | | | | 3 | 3,5 |
| Coco-Caprylate | | | | | | 3 | | |
| Isopropyl Palmitate | 1 | | | | | | 2 | |
| Bis-Ethylhexyloxyphenol Methoxyphenyl Triazin | 2 | 1 | | 2,5 | 0,5 | 3 | | |
| Butyl Methoxydibenzoylmethan | 4,5 | 3 | 3,5 | 4 | 4,5 | 4 | 5 | 3 |
| Diethylamino Hydroxybenzoyl Hexyl Benzoat | | 2 | | | | | | 1 |
| Ethylhexyltriazone | | | | 1 | 0,5 | | | |
| Homosalat | 6 | 4 | 10 | 5 | 7 | 9 | 2 | 8 |
| Octocrylen | 5 | | 8 | 6,5 | | 6 | 9 | 10 |
| Ethylhexylsalicylat | 5 | 3 | 4,5 | 3 | 3,5 | 4 | 2 | 2,5 |
| Polysilicon-15 | | 2 | | | | 3 | | |
| Phenylbenzimidazol Sulfonsäure | 1 | | 1,5 | | 2,5 | | 2 | |
| Titandioxid | 2 | | 4 | 2 | 1,5 | | | |
| Tris-(biphenyl)-1,3,5 triazin | | | 2 | | | | | |
| Tocopherol Acetate | 0,5 | 1 | 0,5 | 0,5 | 1 | 0,5 | 1 | 0,5 |
| Glycerin | 0,5 | 6 | 10 | 9 | 8 | 1 | 2 | 4 |
| Glycyrrhetinsäure | 0,1 | | | | | | | |
| Ubiquinone | | 0,3 | | | | | | |
| Parfum | | 0,2 | 0,8 | 0,7 | 0,5 | 0,3 | 0,4 | |
| Ethylparaben | | | | | | 0,2 | | 0,1 |
| Methylparaben | 0,3 | | | 0,2 | | | 0,3 | 0,2 |
| Phenoxyethanol | | 0,2 | 0,6 | 0,5 | 0,1 | | 0,6 | 0,4 |
| Alcohol Denat. | 6 | 8 | 4 | 9 | 10 | 7 | 6 | 5 |
| EDTA | 1 | 1 | 1 | 1 | 1 | 1 | 1 | 1 |
| Neutralisationsmittel (z.B. Sodium Hydroxide) | q.s. | q.s. | q.s. | q.s. | q.s. | q.s | q.s | q.s |
| Wasser | ad 100 | ad 100 | ad 100 | ad 100 | ad 100 | ad 100 | ad 100 | ad 100 |

## Patentansprüche

1. Kosmetische Öl-in-Wasser Emulsion (O/W Emulsion) enthaltend
a) 4-(tert.-Butyl)-4'-methoxydibenzoylmethan in einer Gesamtkonzentration von 2 bis 5 Gewichts-%, bezogen auf das Gesamtgewicht der Zubereitung,
b) Ethylhexylsalicylat in einer Gesamtkonzentration von 2 bis 5 Gewichts-%, bezogen auf das Gesamtgewicht der Zubereitung,
c) Homomenthylsalicylat (INCI: Homosalate) in einer Gesamtkonzentration von 3 bis 10 Gewichts-%, bezogen auf das Gesamtgewicht der Zubereitung und
d) Natriumstearoylglutamat (INCI Sodium Stearoyl Glutamate).

2. Emulsion nach Anspruch 1, **dadurch gekennzeichnet, dass** die Zubereitung Natriumstearoylglutamat (INCI Sodium Stearoyl Glutamate) in einer Gesamtkonzentration von 0,15 bis 0,75 Gewichts-%, bezogen auf das Gesamtgewicht der Zubereitung, enthält.

3. Emulsion nach einem der vorhergehenden Ansprüche, **dadurch gekennzeichnet, dass** die Zubereitung Ethanol enthält.

4. Emulsion nach einem der vorhergehenden Ansprüche, **dadurch gekennzeichnet, dass** der oder die erfindungsgemäßen Parfümstoffe gewählt werden aus der Gruppe Linalool, Benzyl Benzoate, Hydroxyisohexyl 3-Cyclohexene Carboxaldehyd, Hexyl Cinnamal, Benzyl Salicylate, Citronellol, Coumarin, Limonene, Butylphenyl Methylpropional, Eugenol, Geraniol, Alpha-Isomethyl Ionone.

5. Emulsion nach einem der vorhergehenden Ansprüche, **dadurch gekennzeichnet, dass** die Zubereitung Xanthangummi und/oder Tapiokastärke enthält.

6. Emulsion nach einem der vorhergehenden Ansprüche, **dadurch gekennzeichnet, dass** die Zubereitung ein oder mehrere Diole gewählt aus der Gruppe der Verbindungen 1,2-Pentandiol, 1,2-Hexandiol, 1,2-Octandiol, 1,2-Decandiol, 2-Methylpropan-1,3-diol enthält.

7. Emulsion nach einem der vorhergehenden Ansprüche, **dadurch gekennzeichnet, dass** die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-Ethylhexyl-2-hydroxybenzoat;; Dimethicodiethylbenzalmalonat; 3-(4-(2,2-bis Ethoxycarbonylvinyl)-phenoxy)propenyl)-methoxysiloxan / Dimethylsiloxan - Copolymer; ) 2-Ethylhexyl-2-cyano-3,3-diphenylacrylat (Octocrylen); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

8. Emulsion nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, ß-Alanin und/oder Licochalcon A, enthält.

9. Emulsion nach einem der vorhergehenden Ansprüche, **dadurch gekennzeichnet, dass** die Zubereitung Methylparaben, Ethylparaben, 2-Methyilsothiazol-3(2*H*)-on und/oder Phenoxyethanol enthält.

10. Emulsion nach einem der vorhergehenden Ansprüche, **dadurch gekennzeichnet, dass** die Zubereitung frei ist von 4-Methoxyzimtsäure(2-ethylhexyl)ester (INCI: Ethylhexyl Methoxycinnamate) und p-Methylbenzylidencampher.

11. Emulsion nach einem der vorhergehenden Ansprüche, **dadurch gekennzeichnet, dass** die Zubereitung einen Lichtschutzfaktor (SPF) von größer oder gleich 30 aufweist.

## Claims

1. Cosmetic oil-in-water emulsion (O/W emulsion) comprising
a) 4-(tert-butyl)-4'-methoxydibenzoylmethane at a total concentration of 2 to 5% by weight, based on the total weight of the preparation,
b) ethylhexyl salicylate at a total concentration of 2 to 5% by weight, based on the total weight of the preparation,
c) homomenthyl salicylate (INCI: Homosalate) at a total concentration of 3 to 10% by weight, based on the total weight of the preparation and
d) sodium stearoyl glutamate (INCI: Sodium Stearoyl Glutamate).

2. Emulsion according to Claim 1, **characterized in that** the preparation comprises sodium stearoyl glutamate (INCO Sodium Stearoyl Glutamate) at a total concentration of 0.15 to 0.75% by weight, based on the total weight of the preparation.

3. Emulsion according to either of the preceding claims, **characterized in that** the preparation comprises ethanol.

4. Emulsion according to any of the preceding claims, **characterized in that** the perfume substance(s) according to the invention are selected from the group consisting of linalool, benzyl benzoate, hydroxyisohexyl 3-cyclohexene carboxaldehyde, hexyl cinnamal, benzyl salicylate, citronellol, coumarin, limonene, butylphenyl methylpropional, eugenol, geraniol, alpha-isomethyl ionone.

5. Emulsion according to any of the preceding claims, **characterized in that** the preparation comprises xanthan gum and/or tapioca starch.

6. Emulsion according to any of the preceding claims, **characterized in that** the preparation comprises one or more diols selected from the group of compounds comprising 1,2-pentanediol, 1,2-hexanediol, 1,2-octanediol, 1,2-decanediol, 2-methylpropan-1,3-diol.

7. Emulsion according to any of the preceding claims, **characterized in that** the preparation comprises one or more further UV filters selected from the group of compounds comprising phenylene-1,4-bis(2-benzimidazyl)-3,3'-5,5'-tetrasulphonic acid salts; 2-phenylbenzimidazole-5-sulphonic acid salts; 1,4-di(2-oxo-10-sulpho-3-bornylidenemethyl)benzene and salts thereof; 4-(2-oxo-3-bornylidenemethyl)benzenesulphonic acid salts; 2-methyl-5-(2-oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; hexyl 2-(4'-diethylamino-2'-hydroxybenzoyl)benzoate; terephthalidenedicamphorsulphonic acid; 2-ethylhexyl 4-(dimethylamino)benzoate; amyl 4-(dimethylamino)benzoate; di(2-ethylhexyl) 4-methoxybenzalmalonate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; 2-ethylhexyl 2-hydroxybenzoate; dimethicodiethylbenzalmalonate; 3-(4-(2,2-bisethoxycarbonylvinyl)phenoxy)propenyl)methoxysiloxane/dimethylsiloxan e - copolymer; 2-ethylhexyl 2-cyano-3,3-diphenylacrylate (octocrylene); dioctylbutylamidotriazone (INCI: Diethylhexyl Butamidotriazone); 2,4-bis[5-1(dimethylpropyl)benzoxazol-2-yl-(4-phenyl)imino]-6-(2-ethylhexyl)imino-1,3,5-triazine with (CAS No. 288254-16-0); tris(2-ethylhexyl) 4,4',4"-(1,3,5-triazine-2,4,6-triyltriimino)trisbenzoate (also: 2,4,6-tris[anilino-(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine (INCI: Bis-ethylhexyloxyphenol Methoxyphenyl Triazine); 2,4,6-tris(biphenyl)-1,3,5-triazine; 2,4-bis(4'-dineopentylaminobenzalmalonate)-6-(4"-butylaminobenzoate)-s-triazine; 4-dicyanomethylene-2,6-dimethyl-1,4-dihydropyridine-N-(ethyloxysulphate ester salt), titanium dioxide, zinc oxide, merocyanine, piperazine derivatives.

8. Emulsion according to any of the preceding claims, **characterized in that** the preparation comprises one or more active ingredients selected from the group of compounds comprising gylcyrrhetic acid, urea, arctiin, alpha-lipoic acid, folic acid, phytoene, D-biotin, coenzyme Q10, alpha-glucosylrutin, carnitine, carnosine, natural and/or synthetic isoflavonoids, glycerylglucose, creatine, creatinine, taurine, β-alanine and/or licochalcone A.

9. Emulsion according to any of the preceding claims, **characterized in that** the preparation comprises methylparaben, ethylparaben, 2-methylisothiazol-3(2*H*)-one and/or phenoxyethanol.

10. Emulsion according to any of the preceding claims, **characterized in that** the preparation is free of 2-ethylhexyl 4-methoxycinnamate (INCI: Ethylhexyl Methoxycinnamate) and p-methylbenzylidene camphor.

11. Emulsion according to any of the preceding claims, **characterized in that** the preparation has a sun protection factor (SPF) of greater than or equal to 30.

## Revendications

1. Émulsion cosmétique huile dans eau (émulsion H/E) contenant :
a) du 4-(tert.-butyl)-4'-méthoxydibenzoylméthane en une concentration totale de 2 à 5 % en poids, par rapport au poids total de la préparation,
b) du salicylate d'éthylhexyle en une concentration totale de 2 à 5 % en poids, par rapport au poids total de la préparation,
c) du salicylate d'homomenthyle (INCI : Homosalate) en une concentration totale de 3 à 10 % en poids, par rapport au poids total de la préparation, et
d) du glutamate de stéaroyle sodique (INCI : Sodium Stearoyl Glutamate).

2. Émulsion selon la revendication 1, **caractérisée en ce que** la préparation contient du glutamate de stéaroyle sodique (INCI : Sodium Stearoyl Glutamate) en une concentration totale de 0,15 à 0,75 % en poids, par rapport au poids total de la préparation.

3. Émulsion selon l'une quelconque des revendications précédentes, **caractérisée en ce que** la préparation contient de l'éthanol.

4. Émulsion selon l'une quelconque des revendications précédentes, **caractérisée en ce que** le ou les parfums selon l'invention sont choisis dans le groupe constitué par le linalool, le benzoate de benzyle, l'hydroxyisohexyl-3-cyclohexène-carboxaldéhyde, l'hexylcinnamal, le salicylate de benzyle, le citronellol, la coumarine, le limonène, le butylphényl-méthylpropional, l'eugénol, le géraniol, l'alpha-isométhyl-ionone.

5. Émulsion selon l'une quelconque des revendications précédentes, **caractérisée en ce que** la préparation contient de la gomme xanthane et/ou de l'amidon de tapioca.

6. Émulsion selon l'une quelconque des revendications précédentes, **caractérisée en ce que** la préparation contient un ou plusieurs diols choisis dans le groupe constitué par les composés 1,2-pentanediol, 1,2-hexanediol, 1,2-octanediol, 1,2-décanediol, 2-méthylpropane-1,3-diol.

7. Émulsion selon l'une quelconque des revendications précédentes, **caractérisée en ce que** la préparation contient un ou plusieurs filtres UV supplémentaires choisis dans le groupe constitué par les composés sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; ester hexylique de l'acide 2-(4'-diéthylamino-2'-hydroxybenzoyl)-benzoïque ; acide téréphtalidène-dicamphre-sulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 2-hydroxybenzoate de 2-éthylhexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; acrylate de 2-éthylhexyl-2-cyano-3,3-diphényle (octocrylène) ; dioctylbutylamidotriazone (INCI : Diethylhexyl-Butamidotriazone) ; 2,4-bis-[5-1 (diméthylpropyl)benzoxazol-2-yl-(4-phényl)-imino]-6-(2-éthylhexyl)-imino-1,3,5-triazine de n° CAS 288254-16-0) ; ester tris(2-éthylhexylique) de l'acide 4,4',4"-(1,3,5-triazin-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tris-(biphényl)-1,3,5-triazine ; 2,4-bis-(4'-di-néopentylaminobenzalmalonate)-6-(4"-butylaminobenzoate)-s-triazine, sel de l'ester de N-(éthyloxysulfate) de 4-dicyanométhylène-2,6-diméthyl-1,4-dihydropyridine, dioxyde de titane, oxyde de zinc, mérocyanine, dérivés de pipérazine.

8. Émulsion selon l'une quelconque des revendications précédentes, **caractérisée en ce que** la préparation contient un ou plusieurs agents actifs choisis dans le groupe constitué par les composés acide glycyrrhizique, urée, arctiine, acide alpha-liponique, acide folique, phytoène, D-biotine, coenzyme Q10, alpha-glucosylrutine, carnitine, carnosine, isoflavonoïdes naturels et/ou synthétiques, glycérylglucose, créatine, créatinine, taurine, β-alanine et/ou licochalcone A.

9. Émulsion selon l'une quelconque des revendications précédentes, **caractérisée en ce que** la préparation contient du méthylparabène, de l'éthylparabène, de la 2-méthylisothiazol-3(2*H*)-one et/ou du phénoxyéthanol.

10. Émulsion selon l'une quelconque des revendications précédentes, **caractérisée en ce que** la préparation est exempte d'ester 2-éthylhexylique de l'acide 4-méthoxycinnamique (INCI : Ethylhexyl Methoxycinnamate) et de p-méthylbenzylidène-camphre.

11. Émulsion selon l'une quelconque des revendications précédentes, **caractérisée en ce que** la préparation présente un facteur de protection solaire (FPS) supérieur ou égal à 30.
